Europäisches Patentamt

European Patent Office

Office européen des brevets

(19)

(11) Publication number: **0 027 217**
**A1**

(12) # EUROPEAN PATENT APPLICATION

(21) Application number: **80106001.3**

(22) Date of filing: **03.10.80**

(51) Int. Cl.³: **C 07 J 9/00**

(30) Priority: **04.10.79 US 81957**

(43) Date of publication of application:
**22.04.81 Bulletin 81/16**

(84) Designated Contracting States:
**BE CH DE FR GB LI NL**

(71) Applicant: **EASTMAN KODAK COMPANY**
**343 State Street**
**Rochester, New York 14650(US)**

(72) Inventor: **Foster, Charles Howard**
**P.O. Box 511**
**Kingsport Tennessee(US)**

(72) Inventor: **Nelan, Donald Royce**

**deceased(US)**

(74) Representative: **Brandes, Jürgen, Dr.**
**Dipl.-Chem. et al,**
**Patentanwälte H. Bartels, Dipl.-Chem. Dr. Brandes**
**Dr.-Ing. Held, Dipl.-Phys. Wolff Thierschstrasse 8**
**D-8000 München 22(DE)**

(54) **Process for dehydrogenation of 3-beta-hydroxy steroids.**

(57) This invention relates to the dehydrogenation of 3-$\beta$-hydroxy steroids, such as a mixture of soy sterols, to form the corresponding $\Delta^4$-3-keto derivatives of the steroids. A supported nickel catalyst is used for the reaction and the two different hydrogen acceptors are used in the reaction. The hydrogen acceptors are a dialkyl ketone, such as methyl ethyl ketone, and an olefin, such as 1-hexene.

EP 0 027 217 A1

# PROCESS FOR DEHYDROGENATION OF 3-β-HYDROXY STEROIDS

This invention relates to the dehydrogenation of 3-β-hydroxy steroids, such as a mixture of soy sterols to form the corresponding $\Delta^4$-3-keto derivatives of the steroids using a supported nickel catalyst for the reaction and two chemically different hydrogen acceptors in the reaction.

The naturally occurring phytosterol components of vegetable oils, such as soy oils are composed of mixtures of phytosterols. These phytosterols can be used in the preparation of pharmaceuticals, such as progesterone, which also can be used to prepare other steroids, such as cortisone. The first step in the preparation of progesterone from soy sterols involves the dehydrogenation of the sterols to form the $\Delta^4$-3-keto derivatives. The 3β-OH of the sterol is dehydrogenated to a ketone with rearrangement of the $\Delta^5$ double bond into conjugation with the ketone. Catalytic dehydrogenation is an economical method for this oxidation-rearrangement process. Catalytic dehydrogenation provides a process for dehydrogenation of $\Delta^5$-sterols to ketones with a simultaneous shift of the $\Delta^5$ double bond to the $\Delta^4$ position in conjugation with the carbonyl group. Raney nickel has been used for this reaction, as disclosed in Chakravarti, Chakravarti, and Mitra, Nature, 193, 1071 (1962) or in the presence of a hydrogen acceptor as disclosed in E. C. Kleiderer, and E. C. Kornfeld, J. Org. Chem., 13, 455 (1948) and Kleiderer, Rice, Conquest and Williams, U.S. Dept. of Commerce, Office of the Publication Board, Report PB 981, 1945.

The reaction disclosed by Kleiderer et al was carried out with Raney nickel as a catalyst and cyclohexanone as a hydrogen acceptor. The reaction was carried out for a period of 24 hours and the yield of product varied from 30 to 80 percent.

In accordance with this invention product yields of 75 percent and higher can be obtained in much shorter reaction times. In this invention 3-β-hydroxy steroids, such as mixtures of soy sterols, are dehydrogenated to form the corresponding $\Delta^4$-3-keto derivatives using a supported nickel catalyst and two different hydrogen acceptors, which are a dialkyl ketone and a 1-olefin. The specific examples that illustrate this invention show that product yields of 75 percent and 85 percent can be obtained in reaction periods of only 4 and 8 hours.

The supported nickel catalyst can be a prereduced nickel on a support such as Kieselguhr, silica or a silica/alumina or other suitable support. A suitable commercial catalyst is Girdler G-65S-RS (United Catalyst Co.). The dehydrogenation is carried out in the presence of at least two different hydrogen acceptors. One of the hydrogen acceptors is a dialkyl ketone and the other hydrogen acceptor is a 1-olefin. Useful dialkyl ketones are, for example, methyl ethyl ketone, dimethyl ketone and diethyl ketone. Useful 1-olefins are for example, 1-butene, 1-pentene, 1-hexene, 1-heptene and 1-octene.

The 1-olefin is employed in an amount less than one equivalent, by weight, of the steroids or soy sterols. Greater amounts of 1-olefin tend to cause a decrease in the dehydrogenation reaction rate. Preferably, the amount of 1-olefin employed is about 20 percent by weight of the steroid or soy sterols.

The amount of dialkyl ketone employed is an amount at least the same as the 1-olefin and preferably about 5 to 10 times the amount. Since the dialkyl ketone is used as both a hydrogen acceptor and solvent for the reaction, an excess amount of the dialkyl ketone is generally used.

The dehydrogenation is preferably carried out at elevated temperatures such as 150°C. to 350°C., preferably 200°C.-300°C. and most preferably 240-260°C. At temperatures lower than 150°C., the dehydrogenation reaction is too slow and above 350°C. there is decomposition of the sterols. Since the dehydrogenation temperature is greater than the boiling point of some of the hydrogen acceptors, the reaction is preferably carried out under pressure in an autoclave or similar pressure vessel. The period of time used for the dehydrogenation depends on the temperature used. However, at temperatures of 200°C.-300°C. the reaction is substantially completed in 8 hours.

The amount of catalyst employed varies with the amount of steroid or soy sterol used and the speed of reaction desired for the dehydrogenation reaction. Generally, an amount of catalyst used can be equal to about 20 to 100 percent, preferably 60 to 100 percent, based on the weight of the steroid or soy sterols to be used.

EXAMPLE 1

Preparation of $\Delta^4$-3-keto derivatives of soy sterols was carried out using 20 g. of mixed soy sterols combined with 150 ml of methyl ethyl ketone and 13 ml of 1-hexene and 10 g. of Girdler G-65S-RS catalyst. After heating at 250°C. for 4 hrs. in an autoclave, the $\Delta^4$-3-keto derivatives of soy sterols were the only product obtained and there was 75% conversion. The $\Delta^4$-3-keto derivatives of soy sterols were then reacted by ozonolysis to form 3-ketodinor-4-cholen-22-aldehyde which can be isolated from the other $\Delta^4$-3-keto derivatives of soy sterols by either chromatography or by treatment with sodium bisulfite and extraction with a suitable organic solvent such as toluene.

EXAMPLE 2

Example 1 was repeated except that the reaction was continued for 8 hours instead of 4 hours. An 85% conversion was obtained and the product was the desired $\Delta^4$-3-ketosteroid mixture (80 parts) and the saturated ketosteroid mixture (7 parts).

EXAMPLE 3

Cholesterol (20 g), methyl ethyl ketone (150 ml), and Girdler G65-S-RS catalyst (10.0 g) and 1-hexene (13 g) were heated in a rocking autoclave at 250°C. for 4 hrs. An analysis by gas chromatography indicated conversion to a mixture of 4-cholesten-3-one (78 parts), cholesterol (4 parts), coprostanone (8 parts), and cholestanone (6 parts). Similar results are obtained when sitosterol is used in place of cholesterol.

EXAMPLE 4 (Comparative Example)

This example shows that replacing the methyl ethyl ketone in Example 1 with toluene or dioxane results in a very low conversion. Example 1 was repeated except toluene or dioxane was substituted for methyl ethyl ketone. Analysis of the products showed only 17-27% conversion to the desired products ($\Delta^4$-3-ketosteroids).

EXAMPLE 5 (Comparative Example)

Example 1 was repeated except without the addition of 1-hexene. Analysis of the product showed a mixture of $\Delta^4$-3-ketosteroids (76%), saturated 3-ketosteroids (18%) and unreacted sterols (6%). This example shows the undesirable saturated byproducts formed in the reaction when the 1-olefin is deleted.

The process of the present invention provides an improved method for the dehydrogenation of steroids and soy sterols to provide $\Delta^4$-3-ketosteroids. These 4-en-3-ones derivatives can be used to provide

0027217

materials useful for preparation of valuable steroids such as the cortical steroids.

Claims:

1.  A process for dehydrogenating 3-β-hydroxy steroids to form the corresponding $\Delta^4$-3-keto derivatives characterized by using supported nickel as a catalyst and a dialkyl ketone and a 1-olefin as hydrogen acceptor.

2.  A process according to Claim 1 wherein the dialkyl ketone is methyl ethyl ketone.

3.  A process according to Claim 1 wherein the 1-olefin is 1-hexene.

**European Patent Office**

**EUROPEAN SEARCH REPORT**

Application number

EP 80 10 6001

## DOCUMENTS CONSIDERED TO BE RELEVANT

| Category | Citation of document with indication, where appropriate, of relevant passages | Relevant to claim |
|---|---|---|
| D | JOURNAL OF ORGANIC CHEMISTRY, vol. XIII, no. 3, May 1948, pages 455-458 Washington D.C., U.S.A. E.C. KLEIDERER et al.: "Raney nickel as an organic oxidation-reduction catalyst" <br><br> * Whole article * <br><br> -- | 1 |
| | CHEMICAL ABSTRACTS, vol. 48, no. 9, 14th May 1954, page 5202, column 2, no. 5202e Columbus, Ohio, U.S.A. J. ROMO: "Catalytic oxidations with Raney nickel" <br><br> & BOL. INST. QUIM. UNIV. NACL. AUTON. MEX. 4, 91-100, 1952 <br><br> * Abstract * <br><br> -- | 1 |
| A | US - A - 2 890 226 (ARTHUR R. HANZE) <br><br> * Whole patent * <br><br> -- | 1 |
| A | RECUEIL DES TRAVAUX CHIMIQUES DES PAYS BAS, vol. 56, no. 1, January 1937, pages 137-144 Amsterdam, NL. R.V. OPPENAUER: "Eine Methode der Dehydrierung von sekundären Alkoholen zu Ketonen. I. Zur Herstellung von Sterinketonen und Sexualhormonen" <br><br> * Whole article * <br><br> ---- | 1 |

### CLASSIFICATION OF THE APPLICATION (Int Cl.³)

C 07 J 9/00

### TECHNICAL FIELDS SEARCHED (Int. Cl.³)

C 07 J 9/00

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-12-1980 | HENRY |

EPO Form 1503.1  06.78